# EUROPEAN PATENT SPECIFICATION

(11) **EP 4 122 527 B1**
(45) Date of publication and mention of the grant of the patent: **08.11.2023**
(21) Application number: 21186646.2
(22) Date of filing: 20.07.2021
(51) Int. Cl.: A61N 1/05, H01R 4/20

(54) **METHOD FOR FABRICATING A MEDICAL ELECTRODE DEVICE**
VERFAHREN ZUR HERSTELLUNG EINER MEDIZINISCHEN ELEKTRODENVORRICHTUNG
PROCÉDÉ DE FABRICATION DE DISPOSITIF D'ÉLECTRODE MÉDICALE

(43) Date of publication of application: 25.01.2023
(73) Proprietor: BIOTRONIK SE & Co. KG, 12359 Berlin (DE)
(72) Inventor: Rump, Jens, 12049 Berlin (DE); Buchner, Dagmar, 10245 Berlin (DE); Täubert, Kerstin, 12589 Berlin (DE)
(74) Representative: Biotronik Corporate Services SE

(56) References cited:
- US-A1- 2004 260 310
- US-A1- 2010 179 627
- US-B1- 6 366 820

## Description

The present invention relates to a method for fabricating a medical electrode device for implantation into a patient and to a medical electrode device.

A medical electrode device of this kind may for example serve for a neuro-stimulation and for this may be implanted into a patient for example in the region of the spinal cord, for example into the epidural space near the spinal cord of the spinal column of a patient. In this way a nerve stimulation at the spinal cord may be achieved by injecting electrical stimulation currents.

An electrode device of the kind concerned herein however may also be used for emitting stimulation signals or receiving sense signals at other locations within a patient, for example within the brain or in cardiac applications.

An electrode device for example comprises a lead body extending along a longitudinal axis and having a proximal end and a distal end. An electrode end is arranged at the distal end of the lead body and comprises a carrier element and an arrangement of contact elements arranged on the carrier element for contacting tissue. In an implanted state, the lead body with its proximal end is connected to a generator for generating stimulation signals. The electrode end is implanted in a patient, for example in the epidural space of the spinal column, such that the contact elements of the electrode end are in contact with surrounding tissue and may be used to inject stimulation signals into the tissue in order to provide for a stimulation action in the vicinity e.g. of the spinal cord.

The medical electrode device for example has the shape of a so-called paddle electrode, the electrode end having a paddle-like shape carrying e.g. an arrangement of multiple evenly or unevenly distributed contact elements on its surface for emission of electrical signals into and/or reception of electrical signals from surrounding tissue.

Conventionally, two kinds of electrode devices for a spinal neuro-stimulation exist. Within isodiametric electrodes, contact elements are arranged on a distal portion of the electrode device, the portion of the isodiametric electrode carrying the contact elements having the same diameter as other portions of the electrode device. This allows to percutaneously implant the electrode device using an access to the epidural space in the region of the lumbar spine. For implantation, the electrode device is inserted through vertebral bodies of the spine, and the electrode device is moved in the epidural space towards a region of interest at which a therapy shall be performed. In contrast, a paddle electrode is typically implanted in a patient by surgically placing the electrode end of the electrode device at the location of interest.

US 6,052,608 discloses an electrode device used in particular for sensing cortical electrical activity. The electrode device herein comprises an arrangement of contact elements having a semi-spherical shape, the contact elements being surrounded by an electrically insulating material of a carrier element.

Different designs of paddle electrodes are known, for example, from US 6,895,283, US 2008/0046050 A1, US 2014/0172057 A1 and US 9,561,363.

For a medical electrode device such as a paddle electrode, contact elements are typically formed and placed on a carrier element of an electrode end such that the contact elements are embedded within the material of the electrode end. Supply lines herein are connected to the contact elements in order to provide for an electrical connection of the contact elements, wherein the supply lines typically are connected to the contact elements by forming soldiering connections. Establishing such connections may be cumbersome and hence adds to the cost for fabricating the electrode device.

US 2004/0260310 A1 discloses a medical lead for electrical stimulation or sensing. The lead comprises a generally flat paddle on the distal end of the lead body. An electrode array is provided on the paddle. In an embodiment, herein, a supply line is connected to a contact element using an additional crimp element, the crimp element being connected to the contact element by welding.

It is an object of the instant invention to provide a method for fabricating a medical electrode device and a medical electrode device which allow for an easy and cost-efficient production of the electrode device.

This object is achieved by means of a method for fabricating a medical electrode device comprising the features of claim 1.

Accordingly, a method for fabricating a medical electrode device for implantation into a patient comprises: providing a sleeve element having an inner lumen; inserting an electrical supply line into the lumen of the sleeve element; and deforming said sleeve element to obtain a contact element for the medical electrode device, a portion of the sleeve element forming a contact face of the contact element for electrically contacting tissue, wherein as a result of deforming the sleeve element a crimp connection between the contact element and the electrical supply line is established.

According to the method, a sleeve element is provided for forming the contact element. The sleeve element, in a non-deformed, original state may for example have a cylindrical shape, the lumen longitudinally extending through the sleeve element such that the electrical supply line may be inserted into the lumen for establishing a connection in between the supply line and the sleeve element. For forming the contact element, the sleeve element is plastically deformed, wherein by the deforming a crimp connection in between the contact element and the electrical supply line is established. The contact element herein is formed by the sleeve element itself, a portion of the sleeve element forming a contact face of the contact element which, in an implanted state of the electrode device, serves to contact tissue.

Because the contact element is formed from the sleeve element and because, when forming the contact element, the supply line is immediately connected to the contact element, an easy and cost-efficient fabrication of contact elements and hence of an electrode device can be achieved.

In particular, no additional elements or steps for establishing a connection in between the supply line and the contact element are required. When forming the contact element from the sleeve element, the supply line is immediately connected to the contact element such that the forming of the contact element and the connection of the supply line can be achieved simultaneously in a single step during fabrication.

As no additional soldiering step four connecting the supply line to the contact element is necessary, the fabrication may be eased. In addition, because generally an electrical insulation of the supply line does not have to be removed from the supply line for establishing an electrical connection during the crimping, also a step for removing an insulating cover may no longer be required.

In one embodiment, the sleeve element, prior to the deforming, comprises a cylindrical shape, the lumen extending through the sleeve element. The sleeve element hence may comprise the shape of a tube section, wherein the sleeve element is formed from an electrically conductive material, in particular a metal material, such as a platinum iridium material.

In one embodiment, the deforming step includes: compressing the sleeve element such that the lumen is effectively eliminated. For connecting the electrical supply line to the contact element, the supply line is introduced into the lumen of the sleeve element. By compressing the sleeve element, then, the crimp connection in between the supply line and the contact element is established, wherein by deforming the sleeve element also the contact element may be brought into a desired shape.

In one embodiment, as a result of the deforming, the contact face comprises a convex curvature. Generally, the deforming of the sleeve element may take place in a single step or in multiple steps.

For example, in a first step the sleeve element may be compressed such that a generally flat element is formed from the sleeve element. In a second step, then, the contact element may be formed from the generally flat element, for example by employing a deep-drawing technique or the like, to obtain a three-dimensionally curved contact element.

In another embodiment, the contact element is formed from the sleeve element in a single deformation step, wherein during the deforming the sleeve element is compressed to establish a crimp connection with the electrical supply line and to at the same time form the contact element to assume a desired (three-dimensional) shape.

A convex curvature at the contact face of the contact element may in particular be beneficial for neuro-stimulation applications, a convex curvature allowing for a reliable, efficient contacting of tissue surrounding the electrode device in an implanted state.

In one embodiment, the deforming includes: forming at least one claw portion of the contact element for engaging with a carrier element of the electrode device. By means of such a claw portion the fastening of the contact element on material of the carrier element of the electrode device may be improved in that the claw portion engages with the material of the carrier element and in this way forms a positive-locking or force-locking connection of the contact element to the carrier element.

Claw portions may for example be formed at corners of the contact element, wherein for example four claw portions may be provided at the four corners of a generally rectangular contact element.

In one embodiment, the step of deforming includes: forming at least one depression groove on the contact element for interacting with the electrical supply line. For establishing the connection of the electrical supply line to the contact element the supply line is introduced into the lumen of the sleeve element prior to deforming the sleeve element. The lumen herein extends longitudinally through the sleeve element, wherein the lumen substantially is eliminated by deforming the sleeve element. In order to improve the axial fixation of the supply line within the contact element, herein, a depression groove may be formed on the contact element, the depression groove for example extending transversely to the longitudinal direction and hence transversely to the supply line received in the contact element. By means of the depression groove, hence, an axial fixation may be established, wherein by means of the depression groove in addition the electrical contacting of the supply line may be reliably established.

In one embodiment, the step of inserting the supply line into the sleeve element includes: inserting the electrical supply line into the lumen of the sleeve element with a portion carrying an electrically insulating cover, wherein as a result of the deforming an electrical connection between the contact element and the electrical supply line is established. When forming a crimp connection with the supply line when deforming the sleeve element, the material of the sleeve element cuts through the electrically insulating cover of the supply line, such that an electrically conductive core of the supply line is electrically contacted and hence an electrical connection in between the contact element and the supply line is established. As no further measures are required for removing the electrically insulating cover from the supply line, the fabrication becomes easy in particular in terms of connecting the supply line to the contact element.

In one embodiment, the contact element is placed on a carrier element of the medical electrode device after the sleeve element is deformed and hence the contact element is formed. The contact element hence is placed on and fastened to the carrier element after forming the contact element from the sleeve element. When placing the contact element on the carrier element, the contact element for example may be embedded in the material of the carrier element, for example by pressing the contact element into the material of the carrier element.

To embed the contact element in the material of the carrier element, herein, the contact element may for example be heated, for example to a temperature above 150°C, for example above 180°C, for example at 190°C, wherein upon heating the contact element the contact element is placed on the carrier element and is pressed into the carrier element in order to embed the contact element in the material of the carrier element.

In one embodiment, after placing the contact element on the carrier element the contact face of the contact element is flush with a face of the carrier element. On the face of the carrier element, herein, a multiplicity of contact elements may be arranged, such that an arrangement of evenly or unevenly spaced contact elements is provided on the face of the carrier element. Via the face of the carrier element, hence, the medical electrode device may be brought into electrical contact with tissue in an implanted state of the electrode device.

In another embodiment, the sleeve element may be placed on a carrier element of the medical electrode device prior to deforming the sleeve element. Herein, as a result of deforming the sleeve element, the contact element is fastened to the carrier element. Hence, according to this embodiment the sleeve element prior to deforming is placed on the carrier element, and the sleeve element is deformed on the carrier element in order to form the contact element.

In one embodiment, for placing the sleeve element on the carrier element, the sleeve element may be heated, for example to a temperature above 150°C, beneficially above 180°C, for example at 190°C, in order to allow for an embedding of the sleeve element in the material of the carrier element.

In another embodiment, the carrier element may comprise a reception opening, wherein the sleeve element is placed in the reception opening of the carrier element and is deformed within the reception opening in order to form the contact element on the carrier element. After deforming the sleeve element, the contact element substantially fills the reception opening and is fastened to the carrier element in the reception opening.

In one embodiment, the carrier element may form a protruding portion which reaches into the reception opening and which engages with the lumen of the sleeve element when placing the sleeve element in the reception opening. When deforming the sleeve element, herein, the protruding portion is compressed with the sleeve element and hence improves the mechanical connection of the contact element to the carrier element.

In one embodiment, the contact face of the contact element is flush with a face of the carrier element after deforming the sleeve element on the carrier element. Again, on the face of the carrier element a multiplicity of contact elements may be arranged for electrically contacting with tissue in an implanted state of the electrode device.

The carrier element may for example be made from an electrically insulating material, for example a thermoplastic material, such as a polyurethane material. The carrier element, in one embodiment, may for example have the shape of a polyurethane foil having a thickness in between 0.1 mm and 1 mm, for example 0.2 mm. The carrier element may have, in a lateral plane of extension, a width between 4 mm to 10 mm, for example 5 mm, and a length in between 50 mm and 80 mm, for example 60 mm.

The sleeve element, prior to the deforming, may for example have a diameter between 1 mm and 2 mm, for example 1.15 mm, and a length in between 2 mm and 5 mm, for example between 3 mm and 4 mm. The sleeve element may have a wall thickness of 0.1 mm and may have a cylindrical tube shape forming the inner lumen to receive the supply line therein.

The supply line may for example be formed by an electrically conductive wire, such as a DFT wire.

In another aspect, a medical electrode device for implantation into a patient comprises: a lead body extending longitudinally along a longitudinal axis; and a flattened electrode end arranged at a distal end of the electrode body and comprising a carrier element and a multiplicity of contact elements arranged on the carrier element, wherein at least one of the contact elements is formed by crimping from a sleeve element, an electrical supply line being inserted into the sleeve element and being connected to the contact element by a crimp connection formed by the sleeve element, wherein a portion of the sleeve element forms a contact face of the contact element for electrically contacting tissue.

The medical electrode device beneficially is fabricated using a method as described above.

The advantages and advantageous embodiments described above for the method equally apply also to the medical electrode device.

The contact element hence is formed from a sleeve element, wherein after deforming the sleeve element portions of the sleeve element establish a crimp connection in between the contact element and the electrical supply line. The supply line hence is received in between portions of the contact element, wherein by forming the crimp connection at the same time an electrical connection in between the supply line and the contact element is established.

The various features and advantages of the present invention may be more readily understood with reference to the following detailed description and the embodiments shown in the drawings. Herein,
- Fig. 1: shows a view of an electrode device connected to a stimulation device in an implanted state in the area of the spine of a patient;
- Fig. 2: shows a view of the electrode device in the epidural space in the region of the spinal column;
- Fig. 3: shows a view of an electrode end of an embodiment of an electrode device;
- Fig. 4: shows another view of an electrode end of a medical electrode device;
- Fig. 5A: shows a sleeve element prior to deforming;
- Fig. 5B: shows the sleeve element after deforming for forming a contact element of an electrode device;
- Fig. 6: shows an embodiment of a contact element formed from a sleeve element;
- Fig. 7: shows another embodiment of a contact element formed from a sleeve element;
- Fig. 8: shows an embodiment of a carrier element on which a multiplicity of contact elements are to be placed;
- Fig. 9A: shows a schematic, sectional view of a reception opening formed on the carrier element;
- Fig. 9B: shows the view of Fig. 9A, with a sleeve element placed in the reception opening of the carrier element;
- Fig. 9C: shows the sleeve element of Fig. 9B, with an electrical supply line inserted into a lumen of the sleeve element;
- Fig. 9D: shows the sleeve element of Fig. 9C, after deforming the sleeve element for forming a contact element on the carrier element;
- Fig. 10A: shows a view of another embodiment of a sleeve element on a carrier element;
- Fig. 10B: shows the arrangement of Fig. 10A, with a supply line inserted into the sleeve element;
- Fig. 10C: shows the sleeve element after a deforming step for forming a contact element;
- Fig. 11A: shows the contact element of Fig. 7, prior to placing the contact element on a carrier element; and
- Fig. 11B: shows the contact element, after placing the contact element on the carrier element.

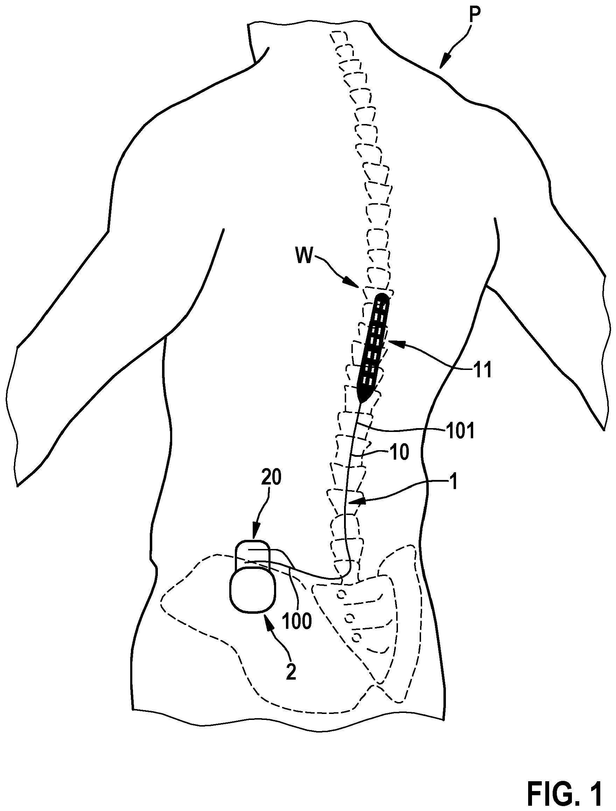

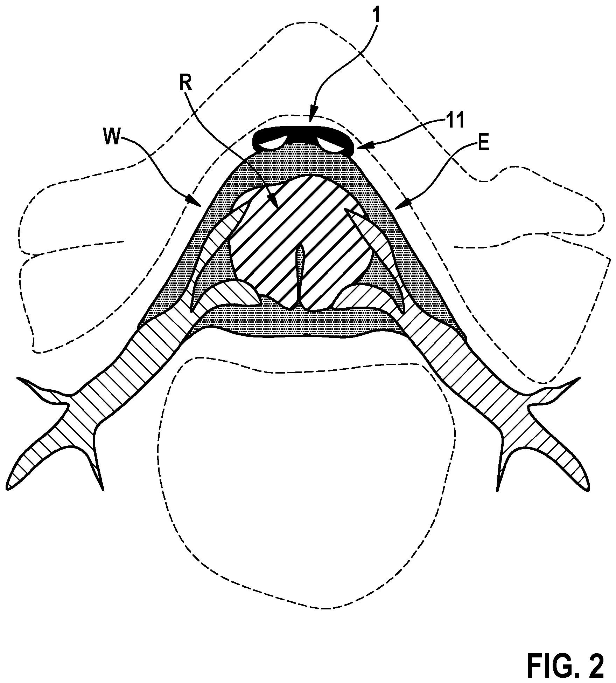

An electrode device 1, as shown in an embodiment in Figs. 1 and 2, is formed as a so-called paddle electrode and comprises a lead body 10 and an electrode end 11 connected to the lead body 10 at a distal end 101 of the lead body 10, a plurality of contact elements being attached to the electrode end 11 for injecting an electrical current e.g. in the region of the spinal column W of a patient P.

The electrode device 1 at a proximal end 100 of the lead body 10 is connected to a connector block 20 of a stimulation device 2, via which stimulation currents can be delivered to the electrode device 1 and output via the electrode arrangement arranged on the electrode end 11 to stimulate the spinal cord R in the region of the spinal column W.

As can be seen from the sectional view of Fig. 2, in the shown embodiment the electrode device 1 is implanted in the epidural space E in the region of the spinal column W of the patient P in such a way that the electrode end 11 is located in the region of the spinal cord R and can thus introduce stimulation currents in a directed manner in order to effect nerve stimulation in the region of the spinal cord R.

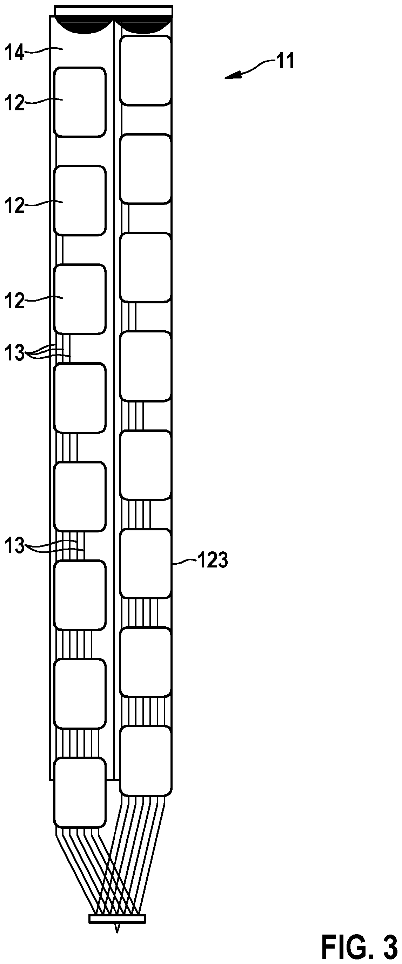

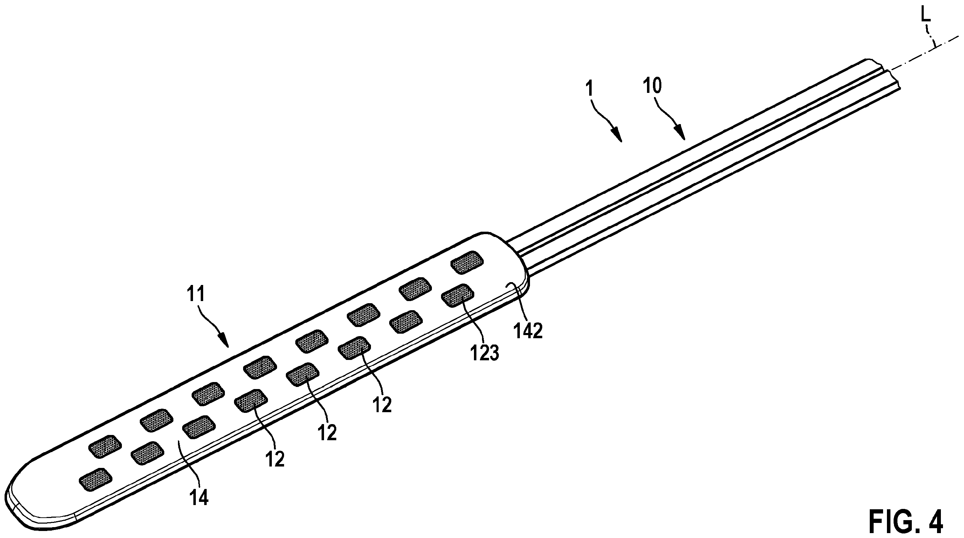

While the lead body 10 for example comprises a circular (isodiametric) cross-section, the electrode device 1 is flattened in the area of the electrode end 11 which, as can be seen in Figs. 3 and 4, carries a plurality of contact elements 12 evenly or unevenly spaced on the electrode end 11 in such a way that stimulation energy can be fed in a directed manner for example into the spinal cord R of a patient P.

As further illustrated in Fig. 3, each contact element 12 is connected to a supply line 13, wherein each contact element 12 for example is connected to the stimulation device 2 via an associated, individual supply line 13 and thus may be supplied with stimulation energy via the stimulation device 2 to emit electrical signals. The supply lines 13 are jointly routed as a single cable strand or, as illustrated in Fig. 4, as multiple cable strands in the lead body 10 in an encapsulated manner to the stimulation device 2.

As shown in Fig. 4, the contact elements 12 are arranged on a carrier element 14 and are exposed, in an implanted state, to tissue with electrical contact faces 123 facing outwards and can therefore come into electrical contact with surrounding tissue when the electrode device 1 is implanted in a patient.

In an electrode device 1 as shown in Figs. 1 to 4, an arrangement of contact elements 12 is arranged on a carrier element 14 of a flattened electrode end 11, the flattened electrode end 11 having a paddle-like shape for placement in the epidural space E in the vicinity of the spinal column W. The contact elements 12 herein are placed on and embedded in the carrier element 14, such that the contact elements 12 each face outwards and are exposed to tissue with their contact face 123.

For fabricating the electrode device 1, it is required to connect the supply lines 13 to the contact elements 12, wherein for example an individual supply line 13 is connected to each contact element 12, as illustrated in Fig. 3. The contact elements 12, with the supply lines 13 connected thereto, are placed on the carrier element 14 to form an arrangement of contact elements 12 for providing for a stimulation and/or sensing in an implanted state of the electrode device 1.

There is a general desire to allow for an easy and cost-efficient fabrication of the electrode device 1.

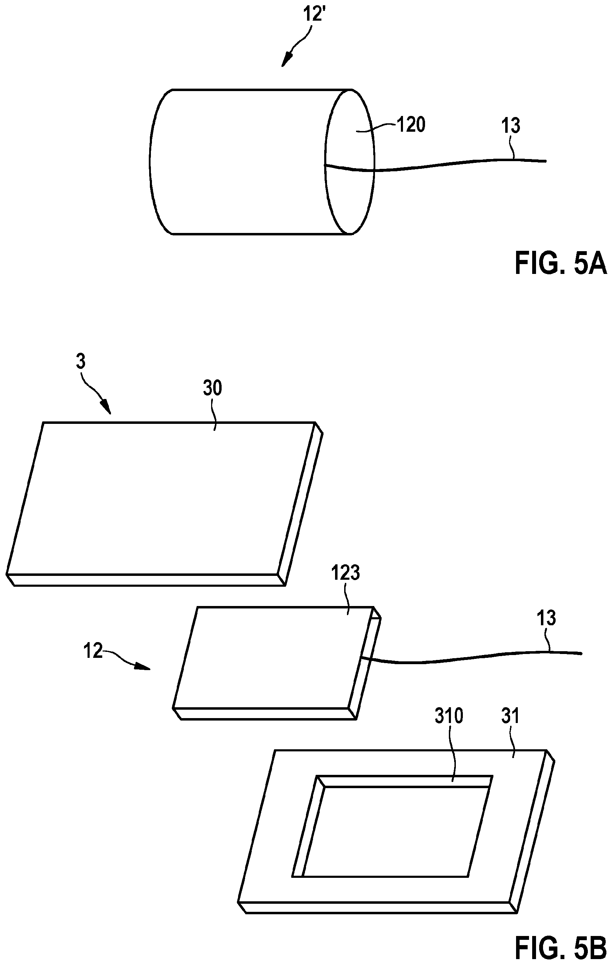

Referring now to Figs. 5A, 5B, in one embodiment a contact element 12 is formed from a sleeve element 12', which in an original, non-deformed state has a cylindrical shape and forms an inner lumen 120 therein. The sleeve element 12' is made from an electrically conductive material, for example a metal material, such as a platinum iridium material, and is deformable to form the contact element 12 and to establish a connection with the supply line 13.

In particular, for forming the contact element 12, the supply line 13 is inserted into the lumen 120 of the sleeve element 12', as shown in Fig. 5A. Subsequently, the sleeve element 12' is deformed by employing a crimp tool 3. The crimp tool 3 may comprise crimp segments 30, 31, which are pressed together with the sleeve element 12' there between to plastically and irrevocably deform the sleeve element 12' for example in a cavity 310 of one of the crimp segments 31 to obtain a plate-like element forming the contact element 12. By deforming the sleeve element 12', the supply line 13 inserted into the lumen 120 of the sleeve element 12' is crimped to the contact element 12, such that the supply line 13 is received and fastened between portions of the sleeve element 12' in its deformed state, and a mechanical as well as an electrical connection in between the supply line 13 and the contact element 12 is established.

Because the connection of the supply length 13 to the contact element 12 is established by crimping, it is not required to remove an electrically insulating cover of the supply line 13 prior to establishing the connection. Rather, when deforming the sleeve element 12' for establishing the crimp connection the material of the sleeve element 12' engages with and cuts through the electrical insulation of the supply line 13 to reliably establish an electrical connection in between the supply line 13 and the contact element 12, in addition to forming a mechanical connection.

The contact element 12 hence is formed by deforming the sleeve element 12'. A portion of the sleeve element 12' herein forms a contact face 123 of the contact element 12, the contact face 123 being shaped to contact tissue when used on an electrode end 11 of the electrode device 1.

The contact element 12 may be arbitrarily shaped from the sleeve element 12'.

In one embodiment, the contact element 12 may assume a substantially two-dimensional, plate-like shape, as shown in Fig. 5B.

In another embodiment, the contact element 12 may assume a curved shape, with a contact face 123 having a convex curvature, such that the contact element 12 is three-dimensionally formed to establish a beneficial contact to surrounding tissue in an implanted state of the electrode device 1.

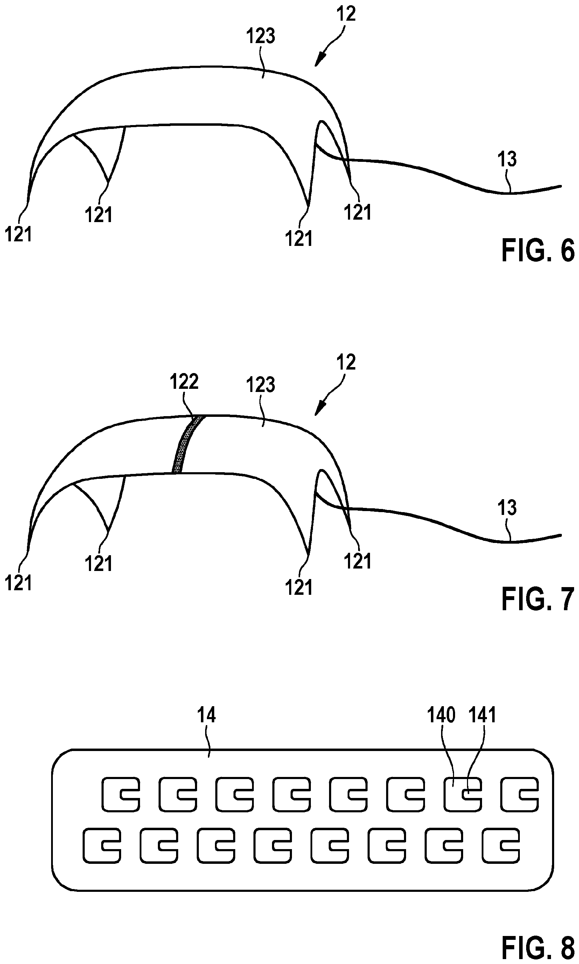

The sleeve element 12' may be formed in a single step to obtain a curved contact element 12 as shown in Fig. 6.

In another embodiment, the sleeve element 12' may in a first step be compressed to establish a crimp connection with the supply line 13 by forming a flat, plate-like element, as shown in Fig. 5B. In another, subsequent step the contact element 12 is formed to assume a three-dimensional, curved shape, as shown in Fig. 6, for example by employing a deep-drawing process.

In the embodiment of Fig. 6, the contact element 12 comprises claw portions 121 at corners of the generally rectangular contact element 12, the claw portions 121 being configured to engage with material of a carrier element 14 for improving the mechanical fastening of the contact element 12 on the carrier element 14.

In another embodiment shown in Fig. 7, a depression groove 122 is formed on the contact face 123 of the contact element 12. The depression groove 122 extends transversely to the supply line 13 and establishes an (additional) axial fixation of the supply line 13 on the contact element 12, wherein the depression groove 122 additionally may improve the electrical connection of the supply line 13 to the contact element 12.

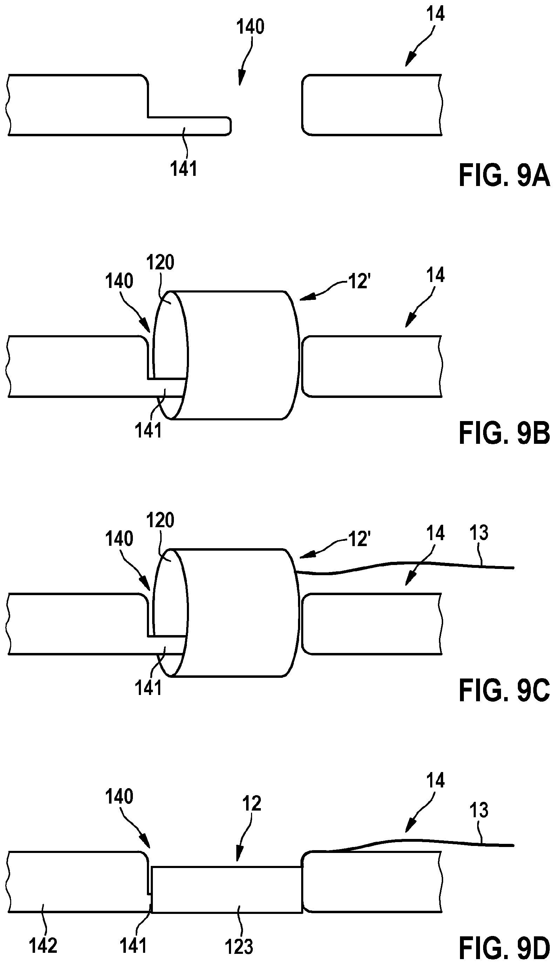

Referring now to Figs. 8 and 9A-9D, in one embodiment the carrier element 14 comprises reception openings 140, into each of which a protruding portion 141 reaches. For forming a contact element 12 on the carrier element 14, a sleeve element 12' is placed in a corresponding reception opening 140, as shown in the transition of Figs. 9A to 9B. The sleeve element 12' may then, upon insertion of a corresponding supply line 13 as shown in Fig. 9C, be deformed to form the contact element 12 immediately on the carrier element 14.

In particular, the contact element 12 may in this way be formed on the carrier element 14 such that the contact face 123 of the contact element 12 serving to contact tissue in an implanted state of the electrode device 1 is flush which a corresponding face 142 of the carrier element 14.

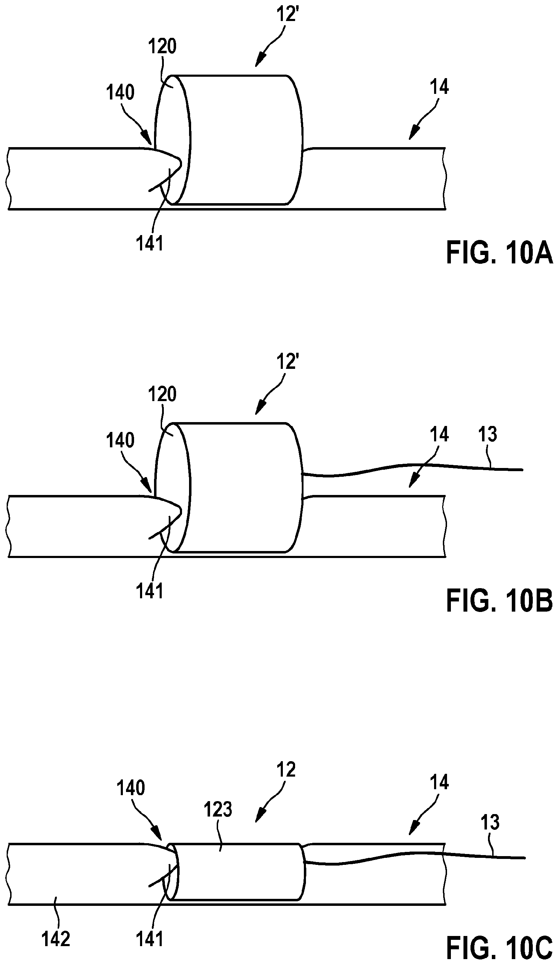

Referring now to Figs. 10A-10C, in another embodiment the sleeve element 12' may be placed on the carrier element 14 by heating the sleeve element 12' to embed the sleeve element 12' in the material of the carrier element 14, as it is shown in Fig. 10A. Upon inserting the corresponding supply line 13 into the lumen 120 of the sleeve element 12' the contact element 12 is formed by deforming the sleeve element 12', as shown in the transition from Fig. 10B to Fig. 10C. Again, the contact element 12 may be formed such that the contact face 123 of the contact element 12 is flush with a corresponding face 142 of the carrier element 14.

In the embodiments of Figs. 8, 9A-9D and Figs. 10A-10C, the supply line 13 is electrically and mechanically connected to the contact element 12 by establishing a crimp connection by deforming the sleeve element 12' to form the contact element 12.

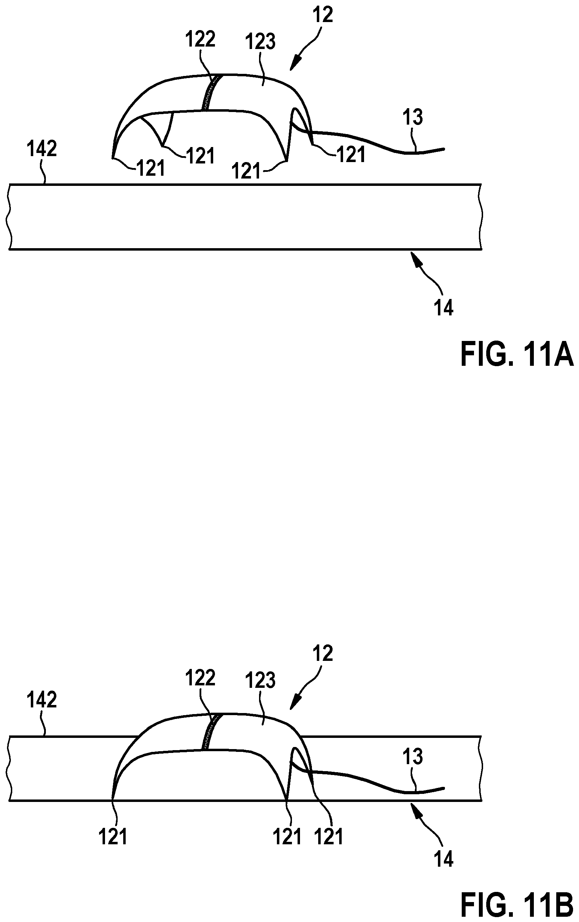

The contact element 12 may be placed on the carrier element 14 by deforming the sleeve element 12' immediately on the carrier element 14, as in the embodiments of Figs. 8 to10A-10C. In another embodiment, the contact element 12 may be formed independent of the carrier element 14 from a sleeve element 12' as shown in Figs. 5A, 5B to 7, wherein subsequently to forming the contact element 12 from the sleeve element 12' the contact element 12 is placed on the carrier element 14 such that it is embedded in the material of the carrier element 14, as it is shown in an example in Figs. 11A, 11B. In a mounted state, as shown in Fig. 11B, the contact element 12 is engaged with the material of the carrier element 14, wherein e.g. claw portions 121 reach into the material of the carrier element 14 to form a secure connection to the carrier element 14. Again, the contact face 123 of the contact element 12 may be flush with the corresponding face 142 of the carrier element 14 or, in another embodiment, may protrude from the face 142.

An electrode device as described herein may in particular be used for a neuro-stimulation in the region of the spinal column of a patient.

An electrode end herein may comprise an arrangement of regularly or irregularly placed contact elements, for example a number of 4 to 32 contact elements, e.g. 16 contact elements, for injecting stimulation signals into tissue or receiving sense signals from tissue.

### List of Reference Numerals

- 1: Implantable electrode device
- 10: Lead body
- 100: Proximal end
- 101: Distal end
- 11: Electrode end
- 12: Contact element
- 12': Sleeve element (pre-state of contact element)
- 120: Opening (lumen)
- 121: Claw portion
- 122: Depression groove
- 123: Contact face
- 13: Supply line
- 14: Carrier element
- 140: Reception opening
- 141: Protruding portion
- 142: Face
- 2: Stimulation device
- 20: Connector block
- 3: Crimp tool
- 30, 31: Crimp segment
- 310: Cavity
- E: Epidural space
- L: Longitudinal axis
- P: Patient
- R: Spinal cord
- W: Spinal column

## Claims

1. A method for fabricating a medical electrode device (1) for implantation into a patient (P), the method comprising:
providing a sleeve element (12') having an inner lumen (120);
inserting an electrical supply line (13) into the lumen (120) of the sleeve element (12'); and
deforming said sleeve element (12') to obtain a contact element (12) for the medical electrode device (1), a portion of the sleeve element (12') forming a contact face (123) of the contact element (12) for electrically contacting tissue, wherein as a result of deforming the sleeve element (12') a crimp connection between the contact element (12) and the electrical supply line (13) is established.

2. The method according to claim 1, wherein the sleeve element (12'), prior to the deforming, comprises a cylindrical shape, the lumen (120) extending through the sleeve element (12').

3. The method according to claim 1 or 2, wherein said deforming includes: compressing the sleeve element (12') such that the lumen (120) is eliminated.

4. The method according to one of claims 1 to 3, wherein as a result of the deforming the contact face (123) comprises a convex curvature.

5. The method according to one of the preceding claims, wherein said deforming includes: forming at least one claw portion (121) on the contact element (12) for engaging with a carrier element (14) of the electrode device (1).

6. The method according to one of the preceding claims, wherein said deforming includes: forming at least one depression groove (122) on the contact element (12) for interacting with the electrical supply line (13).

7. The method according to one of the preceding claims, wherein said inserting includes: inserting the electrical supply line (13) into the lumen (120) of the sleeve element (12') with a portion carrying an electrically insulating cover, wherein as a result of the deforming an electrical connection between the contact element (12) and the electrical supply line (13) is established.

8. The method according to one of claims 1 to 7, wherein after said deforming, placing said contact element (12) on a carrier element (14) of the medical electrode device (1).

9. The method according to claim 8, wherein said placing includes:
heating the contact element (12) to embed the contact element (12) in material of the carrier element (14).

10. The method according to claim 8 or 9, wherein as a result of the placing the contact face (123) of the contact element (12) is flush with a face (142) of the carrier element (14).

11. The method according to one of claims 1 to 7, wherein prior to said deforming, placing the sleeve element (12') on a carrier element (14) of the medical electrode device (1), wherein as a result of the deforming the contact element (12) is fastened to the carrier element (14).

12. The method according to claim 11, wherein said placing includes:
heating the sleeve element (12') to embed the sleeve element (12') in material of the carrier element (14).

13. The method according to claim 11, wherein said placing includes:
placing the sleeve element (12') in a reception opening (140) of the carrier element (14), wherein as a result of the deforming the contact element (12) is fastened in the reception opening (140) of the carrier element (14).

14. The method according to one of claims 11 to 13, wherein as a result of the deforming the contact face (123) of the contact element (12) is flush with a face (142) of the carrier element (14).

15. A medical electrode device (1) for implantation into a patient (P), comprising:
a lead body (10) extending longitudinally along a longitudinal axis (L); and
a flattened electrode end (11) arranged at a distal end of the electrode body (10) and comprising a carrier element (14) and a multiplicity of contact elements (12) arranged on the carrier element (14), wherein at least one of the contact elements (12) is formed by crimping from a sleeve element (12'), an electrical supply line (13) being inserted into the sleeve element (12') and being connected to the contact element (12) by a crimp connection formed by the sleeve element (12'), wherein a portion of the sleeve element (12') forms a contact face (123) of the contact element (12) for electrically contacting tissue.

## Patentansprüche

1. Verfahren zur Herstellung einer medizinischen Elektrodenvorrichtung (1) zur Implantation in einen Patienten (P), wobei das Verfahren umfasst:
Bereitstellung eines Hülsenelements (12') mit einem inneren Lumen (120);
Einführen einer elektrischen Zuleitung (13) in das Lumen (120) des Hülsenelements (12'); und
Verformen des Hülsenelements (12'), um ein Kontaktelement (12) für die medizinische Elektrodenvorrichtung (1) zu erhalten, wobei ein Teil des Hülsenelements (12') eine Kontaktfläche (123) des Kontaktelements (12) zur elektrischen Kontaktierung von Gewebe bildet, wobei als Ergebnis des Verformens des Hülsenelements (12') eine Crimpverbindung zwischen dem Kontaktelement (12) und der elektrischen Zuleitung (13) hergestellt wird.

2. Verfahren nach Anspruch 1, **dadurch gekennzeichnet, dass** das Hülsenelement (12') vor der Verformung eine zylindrische Form aufweist, wobei sich das Lumen (120) durch das Hülsenelement (12') erstreckt.

3. Verfahren nach Anspruch 1 oder 2, **dadurch gekennzeichnet, dass** die Verformung Folgendes umfasst: Zusammendrücken des Hülsenelements (12'), so dass das Lumen (120) beseitigt wird.

4. Verfahren nach einem der Ansprüche 1 bis 3, **dadurch gekennzeichnet, dass** die Kontaktfläche (123) infolge der Verformung eine konvexe Krümmung aufweist.

5. Verfahren nach einem der vorhergehenden Ansprüche, **dadurch gekennzeichnet, dass** die Verformung Folgendes umfasst: Ausbilden mindestens eines Klauenabschnitts (121) auf dem Kontaktelement (12) zum Eingreifen in ein Trägerelement (14) der Elektrodenvorrichtung (1).

6. Verfahren nach einem der vorhergehenden Ansprüche, **dadurch gekennzeichnet, dass** die Verformung Folgendes umfasst: Ausbilden mindestens einer Vertiefungsrille (122) auf dem Kontaktelement (12), die mit der elektrischen Zuleitung (13) zusammenwirkt.

7. Verfahren nach einem der vorhergehenden Ansprüche, **dadurch gekennzeichnet, dass** das Einführen Folgendes umfasst: Einführen der elektrischen Zuleitung (13) in das Lumen (120) des Hülsenelements (12') mit einem Abschnitt, der eine elektrisch isolierende Abdeckung trägt, wobei als Ergebnis der Verformung eine elektrische Verbindung zwischen dem Kontaktelement (12) und der elektrischen Zuleitung (13) hergestellt wird.

8. Verfahren nach einem der Ansprüche 1 bis 7, **dadurch gekennzeichnet, dass** nach der Verformung das Kontaktelement (12) auf ein Trägerelement (14) der medizinischen Elektrodenvorrichtung (1) aufgesetzt wird.

9. Verfahren nach Anspruch 8, **dadurch gekennzeichnet, dass** die Platzierung Folgendes umfasst: Erhitzen des Kontaktelements (12), um das Kontaktelement (12) in das Material des Trägerelements (14) einzubetten.

10. Verfahren nach Anspruch 8 oder 9, **dadurch gekennzeichnet, dass** durch die Platzierung die Kontaktfläche (123) des Kontaktelements (12) mit einer Fläche (142) des Trägerelements (14) bündig ist.

11. Verfahren nach einem der Ansprüche 1 bis 7, **dadurch gekennzeichnet, dass** vor der Verformung das Hülsenelement (12') auf ein Trägerelement (14) der medizinischen Elektrodenvorrichtung (1) aufgesetzt wird, wobei durch die Verformung das Kontaktelement (12) an dem Trägerelement (14) befestigt wird.

12. Verfahren nach Anspruch 11, **dadurch gekennzeichnet, dass** das Anbringen umfasst: Erhitzen des Hülsenelements (12'), um das Hülsenelement (12') in das Material des Trägerelements (14) einzubetten.

13. Verfahren nach Anspruch 11, **dadurch gekennzeichnet, dass** das Platzieren Folgendes umfasst: Platzieren des Hülsenelements (12') in einer Aufnahmeöffnung (140) des Trägerelements (14), wobei als Ergebnis der Verformung das Kontaktelement (12) in der Aufnahmeöffnung (140) des Trägerelements (14) befestigt wird.

14. Verfahren nach einem der Ansprüche 11 bis 13, **dadurch gekennzeichnet, dass** durch die Verformung die Kontaktfläche (123) des Kontaktelements (12) mit einer Fläche (142) des Trägerelements (14) bündig ist.

15. medizinische Elektrodenvorrichtung (1) zur Implantation in einen Patienten (P), umfassend:
einen Leitungskörper (10), der sich in Längsrichtung entlang einer Längsachse (L) erstreckt; und
ein abgeflachtes Elektrodenende (11), das an einem distalen Ende des Elektrodenkörpers (10) angeordnet ist und ein Trägerelement (14) und eine Vielzahl von auf dem Trägerelement (14) angeordneten Kontaktelementen (12) aufweist, wobei mindestens eines der Kontaktelemente (12) durch Crimpen aus einem Hülsenelement (12') gebildet ist, wobei in das Hülsenelement (12') eine elektrische Zuleitung (13) eingelegt ist, die durch eine vom Hülsenelement (12') gebildete Crimpverbindung mit dem Kontaktelement (12) verbunden ist, wobei ein Teil des Hülsenelements (12') eine Kontaktfläche (123) des Kontaktelements (12) zur elektrischen Kontaktierung von Gewebe bildet.

## Revendications

1. Une méthode de fabrication d'un dispositif d'électrode médicale (1) destiné à être implanté dans un patient (P), la méthode comprenant:
fournir un élément de manchon (12') ayant une lumière intérieure (120);
l'insertion d'une ligne d'alimentation électrique (13) dans la lumière (120) de l'élément de manchon (12'); et
déformer ledit élément de manchon (12') pour obtenir un élément de contact (12) pour le dispositif d'électrode médicale (1), une partie de l'élément de manchon (12') formant une face de contact (123) de l'élément de contact (12) pour le contact électrique avec le tissu, dans lequel, à la suite de la déformation de l'élément de manchon (12'), une connexion par sertissage entre l'élément de contact (12) et la ligne d'alimentation électrique (13) est établie.

2. La méthode selon la revendication 1, **caractérisée en ce que** l'élément de manchon (12'), avant la déformation, comprend une forme cylindrique, la lumière (120) s'étendant à travers l'élément de manchon (12').

3. La méthode selon la revendication 1 ou 2, **caractérisé en ce que** ladite déformation comprend: la compression de l'élément de manchon (12') de manière à éliminer la lumière (120).

4. La méthode selon l'une des revendications 1 à 3, **caractérisé en ce que**, suite à la déformation, la face de contact (123) présente une courbure convexe.

5. La méthode selon l'une des revendications précédentes, **caractérisé en ce que** ladite déformation comprend: la formation d'au moins une partie en forme de griffe (121) sur l'élément de contact (12) pour s'engager avec un élément porteur (14) du dispositif d'électrodes (1).

6. La méthode selon l'une des revendications précédentes, **caractérisé en ce que** ladite déformation comprend: la formation d'au moins une rainure de dépression (122) sur l'élément de contact (12) pour interagir avec la ligne d'alimentation électrique (13).

7. La méthode selon l'une des revendications précédentes, **caractérisé en ce que** ladite insertion comprend: l'insertion de la ligne d'alimentation électrique (13) dans la lumière (120) de l'élément de manchon (12') avec une partie portant une couverture électriquement isolante, où, à la suite de la déformation, une connexion électrique entre l'élément de contact (12) et la ligne d'alimentation électrique (13) est établie.

8. La méthode selon l'une des revendications 1 à 7, **caractérisé par:** après ladite déformation, placer ledit élément de contact (12) sur un élément porteur (14) du dispositif d'électrodes médicales (1).

9. La méthode selon la revendication 8, **caractérisé en ce que** la mise en place comprend: le chauffage de l'élément de contact (12) pour encastrer l'élément de contact (12) dans le matériau de l'élément porteur (14).

10. La méthode selon la revendication 8 ou 9, **caractérisé en ce qu'**à la suite de la mise en place, la face de contact (123) de l'élément de contact (12) affleure une face (142) de l'élément porteur (14).

11. La méthode selon l'une des revendications 1 à 7, **caractérisée par:** avant ladite déformation, le placement de l'élément de manchon (12') sur un élément porteur (14) du dispositif d'électrode médicale (1), où, à la suite de la déformation, l'élément de contact (12) est fixé à l'élément porteur (14).

12. La méthode selon la revendication 11, **caractérisée en ce que** la mise en place comprend: le chauffage de l'élément de manchon (12') pour encastrer l'élément de manchon (12') dans le matériau de l'élément de support (14).

13. La méthode selon la revendication 11, **caractérisée en ce que** la mise en place comprend: la mise en place de l'élément de manchon (12') dans une ouverture de réception (140) de l'élément porteur (14), où, à la suite de la déformation, l'élément de contact (12) est fixé dans l'ouverture de réception (140) de l'élément porteur (14).

14. La méthode selon l'une des revendications 11 à 13, **caractérisé en ce qu'**à la suite de la déformation, la face de contact (123) de l'élément de contact (12) affleure une face (142) de l'élément porteur (14).

15. Dispositif d'électrode médicale (1) à implanter dans un patient (P), comprenant :
un corps de conducteur (10) s'étendant longitudinalement le long d'un axe longitudinal (L); et
une extrémité d'électrode aplatie (11) disposée à une extrémité distale du corps d'électrode (10) et comprenant un élément porteur (14) et une multiplicité d'éléments de contact (12) disposés sur l'élément porteur (14), dans lequel au moins un des éléments de contact (12) est formé par sertissage à partir d'un élément de manchon (12'), une ligne d'alimentation électrique (13) est insérée dans l'élément de manchon (12') et est connectée à l'élément de contact (12) par une connexion sertie formée par l'élément de manchon (12'), une partie de l'élément de manchon (12') formant une face de contact (123) de l'élément de contact (12) pour le contact électrique avec le tissu.
